# EUROPEAN PATENT APPLICATION

(11) **EP 4 825 366 A1**
(43) Date of publication of application: **21.10.2026**
(21) Application number: 24903519.7
(22) Date of filing: 29.11.2024
(51) Int. Cl.: A61K 8/37, A61K 8/42, A61K 8/73, A61K 8/87, A61K 8/88, A61K 8/92, A61Q 17/04

(54) **COSMETIC COMPOSITION**

(30) Priority: 15.12.2023 JP 2023212476
(71) Applicant: Shiseido Company, Ltd., Chuo-ku Tokyo 104-0061 (JP)
(72) Inventor: UEHARA, Makoto, Tokyo 104-0061 (JP); INUKAI, Daiki, Tokyo 104-0061 (JP); TOMITA, Noriko, Tokyo 104-0061 (JP)
(74) Representative: Ter Meer und Partner mbB
(86) International application number: PCT/JP2024/042258
(87) International publication number: WO 2025/126861

(57) **Abstract**

Provided is a cosmetic composition having satisfactory usability.

A cosmetic composition, comprising
a compound I having a structure of formula (I) below: 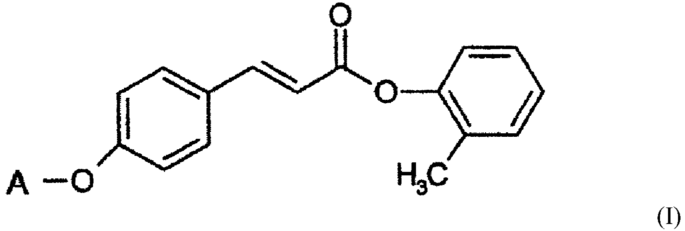 where in the formula, -OA represents an alkoxy group;
an oil component comprising a liquid polar oil; and
an oil-phase thickener, wherein
the compound I is dissolved in the oil component,
the compound I, relative to 100 parts by mass of the liquid polar oil, is 2.8 parts by mass or more, and
the oil-phase thickener comprises at least one selected from the group consisting of waxes and oil gelling agents having a saccharide backbone, an amide bond, or a urethane bond.

## Description

### FIELD

The present invention relates to a cosmetic composition.

### BACKGROUND

Protecting the skin from ultraviolet rays is an important problem in skin care and body care. Not only mid-wavelength ultraviolet rays (UVB region: wavelength of 290 to 320 nm), which are known to cause sunburn and inflammation, but also long-wavelength ultraviolet rays (UVA region: wavelength of 320 to 400 nm) are known to have effects on the skin (such as photoaging).

To date, for example, PTL 1 discloses a compound, as an ultraviolet absorber having absorption capacity in both the UVA region and the UVB region, represented by the following general formula I: 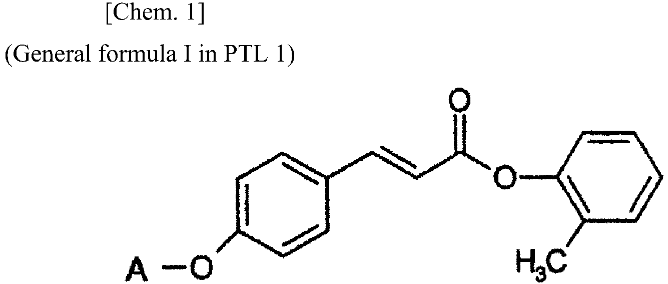 where in the formula, -OA represents an alkoxy group.

### [CITATION LIST]

### [PATENT LITERATURE]

[PTL 1] WO 2009/041098

### SUMMARY

### [TECHNICAL PROBLEM]

The present inventors have discovered through intensive research that when the compound represented by the above general formula I is blended into an oil component and used, crystals of the compound represented by the general formula I may precipitate over time. When crystals precipitate from a cosmetic, grittiness may be perceived at the time of application, resulting in poor usability.

To address the issue of poor usability due to such crystallization, for example, measures such as blending a relatively large amount of polar oil relative to the compound represented by the above general formula I have been considered.

However, as the content of polar oil in a cosmetic increases, tackiness may be perceived.

The present invention aims to improve upon the above circumstances, and has an object of providing a cosmetic composition having satisfactory usability.

### [SOLUTION TO PROBLEM]

The present invention for achieving the above object is as follows.

### <Aspect 1>

A cosmetic composition, comprising
a compound I having a structure of formula (I) below: 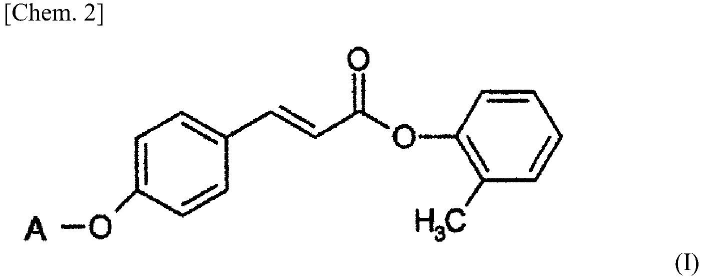 where in the formula, -OA represents an alkoxy group;
an oil component comprising a liquid polar oil; and
an oil-phase thickener,
wherein the compound I is dissolved in the oil component,
wherein the compound I, relative to 100 parts by mass of the liquid polar oil, is 2.8 parts by mass or more, and
wherein the oil-phase thickener comprises at least one selected from a group consisting of waxes and oil gelling agents having a saccharide backbone, an amide bond, or a urethane bond.

### <Aspect 2>

The cosmetic composition according to Aspect 1, wherein the wax is at least one selected from a group consisting of microcrystalline wax, polyethylene, candelilla wax, sunflower seed wax, paraffin, synthetic wax, beeswax, Japan wax, rice bran wax, carnauba wax, and ceresin.

### <Aspect 3>

The cosmetic composition according to Aspect 1 or 2, wherein the oil gelling agent is at least one selected from a group consisting of dibutyl lauroyl glutamide, polyamide-8, polyurethane-79, dextrin palmitate, dextrin (palmitate/ethylhexanoate), dibutyl ethylhexanoyl glutamide, and dextrin myristate.

### <Aspect 4>

The cosmetic composition according to any one of Aspects 1 to 3, wherein the oil-phase thickener, relative to 100 parts by mass in total of the compound I, the oil component, and the oil-phase thickener, is 0.1 parts by mass or more and 15 parts by mass or less.

### <Aspect 5>

The cosmetic composition according to any one of Aspects 1 to 4, wherein content of the liquid polar oil in the oil component is 10% by mass or more.

### <Aspect 6>

The cosmetic composition according to any one of Aspects 1 to 5, wherein the compound I, relative to 100 parts by mass in total of the compound I, the oil component, and the oil-phase thickener, is 3.0 parts by mass or more.

### <Aspect 7>

An oil-based cosmetic comprising the cosmetic composition according to any one of Aspects 1 to 6.

### <Aspect 8>

An oil-based solid cosmetic comprising the cosmetic composition according to any one of Aspects 1 to 6.

### <Aspect 9>

A water-in-oil emulsion cosmetic comprising the cosmetic composition according to any one of Aspects 1 to 6.

### <Aspect 10>

A sunscreen cosmetic comprising the cosmetic composition according to any one of Aspects 1 to 6.

### [ADVANTAGEOUS EFFECTS OF INVENTION]

According to the present invention, a cosmetic composition having satisfactory usability can be provided.

### DESCRIPTION OF EMBODIMENTS

Hereinafter, embodiments of the present invention will be described in detail. The present invention is not limited to the following embodiments, and various modifications can be made within the scope of the invention.

### <<Cosmetic composition>>

The cosmetic composition of the present invention (hereinafter, also simply referred to as "composition of the present invention") is a cosmetic composition, comprising
a compound I having a structure of formula (I) below: 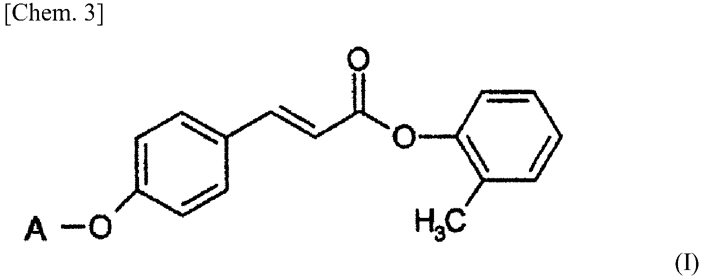 where in the formula, -OA represents an alkoxy group;
an oil component comprising a liquid polar oil; and
an oil-phase thickener, wherein
the compound I is dissolved in the oil component,
the compound I, relative to 100 parts by mass of the liquid polar oil, is 2.8 parts by mass or more, and
the oil-phase thickener comprises at least one selected from a group consisting of waxes and oil gelling agents having a saccharide backbone, an amide bond, or a urethane bond.

The present inventors have discovered through intensive research that in the composition of the present invention, even when the proportion of the compound I relative to the liquid polar oil is comparatively large, crystallization, particularly crystallization over time, of the compound I can be suppressed when a specific oil-phase thickener is used in combination. "Suppressing crystallization over time" refers to an ability to suppress crystallization of the compound I not only immediately after the composition is prepared but also, for example, after standing at room temperature (25°C) for 4 days or preferably even after standing for 1 week. The presence or absence of crystallization of the compound I may be comprehensively determined by visual inspection and application of the composition, and more specifically, the Examples section below can be referenced.

In the present invention, when the proportion of the compound I relative to a liquid polar oil is comparatively large, for example, (i) the proportion of the compound I in the cosmetic composition may be comparatively large, and the proportion of the liquid polar oil in the cosmetic composition may be comparatively small; (ii) when the proportion of the compound I in the cosmetic composition may be comparatively small, and the proportion of the liquid polar oil in the cosmetic composition may also be small; or (iii) when the proportion of the liquid polar oil in the cosmetic composition may be comparatively large, and the proportion of the compound represented by the general formula I in the cosmetic composition may also be large. In the present invention, from the viewpoint of suppressing tackiness, cases (i) and (ii) above are particularly preferable. "Liquid polar oil" in the present specification refers to a polar oil that is liquid at 25°C, and needs only to be in a state in which the compound I can be dissolved when preparing the cosmetic composition.

Without being bound by theory, it is presumed that the mechanism by which crystallization of the compound I can be suppressed in the composition of the present invention is attributed to the fact that, by blending a specific oil-phase thickener, a three-dimensional network structure can be formed to an extent capable of restricting motion of molecules of the compound I. In addition, it is considered that a wax used as an oil-phase thickener contributes to the three-dimensional network structure by adopting a card-house structure, and it is considered that an oil gelling agent having a saccharide backbone, an amide bond, or a urethane bond used as an oil-phase thickener contributes to the three-dimensional network structure by the formation of hydrogen bonds.

Hereinafter, each component that can be contained in the composition of the present invention will be described in detail.

### <Compound I>

The composition of the present invention comprises a compound I. By comprising the compound I, the composition exhibits absorption capacity in both the UVA region and the UVB region and exhibits ultraviolet absorption capabilities in a broad range of wavelengths.

The compound I according to the present invention has a structure of the formula below: 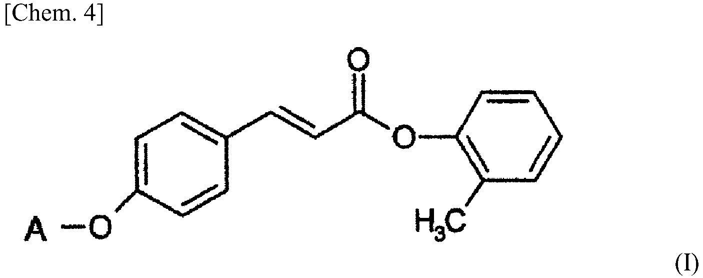 where in the formula, -OA represents an alkoxy group.

In the formula (I), -OA represents an alkoxy group, and more specifically includes, but is not limited to, for example, a methoxy group and an ethoxy group.

In the present invention, the compound I may have the same structure as the compound represented by general formula I disclosed in PTL 1.

In the present invention, the compound I is dissolved in an oil component. In the present invention, the compound I being dissolved in an oil component, when the composition of the present invention and a cosmetic comprising the composition of the present invention are liquid, refers to a state in which 99% by mass or more, preferably 100% by mass, of the compound I is dissolved in a liquid oil component, and when the composition of the present invention and a cosmetic comprising the composition of the present invention are solid, refers to a state in which 99% by mass or more, preferably 100% by mass, of the compound I is dissolved in a liquid oil component when preparing the cosmetic.

In the composition of the present invention, the content of the compound I is not particularly limited. For example, the compound I, relative to 100 parts by mass in total of the compound I, the oil component, and the oil-phase thickener, may be 1.0 parts by mass or more, 1.5 parts by mass or more, 2.0 parts by mass or more, 2.5 parts by mass or more, 3.0 parts by mass or more, 3.1 parts by mass or more, 3.2 parts by mass or more, 3.3 parts by mass or more, 3.4 parts by mass or more, 3.5 parts by mass or more, 3.6 parts by mass or more, 3.7 parts by mass or more, 3.8 parts by mass or more, 3.9 parts by mass or more, 4.0 parts by mass or more, 4.1 parts by mass or more, 4.2 parts by mass or more, 4.3 parts by mass or more, 4.4 parts by mass or more, 4.5 parts by mass or more, 4.6 parts by mass or more, 4.7 parts by mass or more, 4.8 parts by mass or more, 4.9 parts by mass or more, or 5.0 parts by mass or more, and may be 10 parts by mass or less, 9.5 parts by mass or less, 9.0 parts by mass or less, 8.5 parts by mass or less, 8.0 parts by mass or less, 7.5 parts by mass or less, 7.0 parts by mass or less, 6.5 parts by mass or less, 6.0 parts by mass or less, 5.5 parts by mass or less, 5.0 parts by mass or less, 4.5 parts by mass or less, 4.0 parts by mass or less, 3.5 parts by mass or less, or 3.0 parts by mass or less.

In the composition of the present invention, the compound I, relative to 100 parts by mass of the liquid polar oil, may be 2.8 parts by mass or more, 3.0 parts by mass or more, 3.2 parts by mass or more, 3.4 parts by mass or more, 3.6 parts by mass or more, 3.8 parts by mass or more, 4.0 parts by mass or more, 4.2 parts by mass or more, 4.4 parts by mass or more, 4.6 parts by mass or more, 4.8 parts by mass or more, 5.0 parts by mass or more, 5.2 parts by mass or more, 5.4 parts by mass or more, 5.6 parts by mass or more, 5.8 parts by mass or more, 6.0 parts by mass or more, 6.2 parts by mass or more, 6.4 parts by mass or more, 6.6 parts by mass or more, 6.8 parts by mass or more, 7.0 parts by mass or more, 7.2 parts by mass or more, 7.4 parts by mass or more, 7.6 parts by mass or more, 7.8 parts by mass or more, 8.0 parts by mass or more, 8.2 parts by mass or more, 8.4 parts by mass or more, 8.6 parts by mass or more, 8.8 parts by mass or more, 9.0 parts by mass or more, 9.2 parts by mass or more, 9.4 parts by mass or more, 9.6 parts by mass or more, 9.8 parts by mass or more, 10.0 parts by mass or more, 11.0 parts by mass or more, 12.0 parts by mass or more, 13.0 parts by mass or more, 14.0 parts by mass or more, 15.0 parts by mass or more, 16.0 parts by mass or more, 17.0 parts by mass or more, 18.0 parts by mass or more, 19.0 parts by mass or more, 20.0 parts by mass or more, 21.0 parts by mass or more, or 22.0 parts by mass or more, and may be 50.0 parts by mass or less, 45.0 parts by mass or less, 40.0 parts by mass or less, 35.0 parts by mass or less, 30.0 parts by mass or less, 25.0 parts by mass or less, 20.0 parts by mass or less, 19.0 parts by mass or less, 18.0 parts by mass or less, 17.0 parts by mass or less, 16.0 parts by mass or less, 15.0 parts by mass or less, 14.0 parts by mass or less, 13.0 parts by mass or less, or 12.0 parts by mass or less.

### <Oil component>

The composition of the present invention comprises an oil component. The oil component comprises a liquid polar oil.

In the composition of the present invention, the content of the oil component is not particularly limited. For example, the oil component, relative to 100 parts by mass in total of the compound I, the oil component, and the oil-phase thickener, may be 75 parts by mass or more, 76 parts by mass or more, 77 parts by mass or more, 78 parts by mass or more, 79 parts by mass or more, 80 parts by mass or more, 81 parts by mass or more, 82 parts by mass or more, 83 parts by mass or more, 84 parts by mass or more, 85 parts by mass or more, 86 parts by mass or more, 87 parts by mass or more, 88 parts by mass or more, 89 parts by mass or more, 90 parts by mass or more, 91 parts by mass or more, or 92 parts by mass or more, and may be 96.9 parts by mass or less, 96.5 parts by mass or less, 96 parts by mass or less, 95.5 parts by mass or less, 95 parts by mass or less, 94.5 parts by mass or less, 94 parts by mass or less, 93.5 parts by mass or less, 93 parts by mass or less, 92.5 parts by mass or less, or 92 parts by mass or less.

### (Polar oil)

In the present invention, "polar oil" refers to a highly polar oil component, among oil components usable in cosmetics, other than a silicone oil, and refers to a polar oil having, for example, an IOB value of 0.10 or greater, 0.11 or greater, 0.12 or greater, or 0.13 or greater. The IOB value of the polar oil according to the present invention may be 2.0 or less, 1.5 or less, 1.0 or less, 0.50 or less, 0.45 or less, or 0.40 or less. IOB value is an abbreviation for inorganic/organic balance, is a value that represents a ratio of inorganic value to organic value, and acts as an indicator showing the degree of polarity of an organic compound. The IOB value is specifically represented as: IOB value = inorganic value / organic value. With respect to each of "inorganic value" and "organic value," the "inorganic value" and "organic value" are set depending on various atoms or functional groups, such that, for example, the "organic value" for one carbon atom in a molecule is 20, and the "inorganic value" for one hydroxyl group is 100, and by summing the "inorganic values" and "organic values" of all atoms and functional groups in an organic compound, the IOB value of the organic compound can be calculated (refer to, for example, Yoshio Koda, Organic Conceptual Diagrams - Fundamentals and Applications -, pp. 11 to 17, Sankyo Publishing Co., Ltd., published in 1984).

In the present invention, the polar oil may be at least one selected from the group consisting of ester oils, ether oils, higher alcohols, and fatty acids, having an IOB value of 0.10 or greater. Particularly, among these polar oils, a liquid polar oil capable of dissolving the above compound I is preferable.

More specifically, the polar oil, for example, may be at least one selected from the group consisting of polyglyceryl-10 laurate (IOB value = 1.60), PEG-20 sorbitan cocoate (IOB value = 1.49), bis-ethoxydiglycol cyclohexane-1,4-dicarboxylate (IOB value = 1.03), phenoxyethyl caprylate (IOB value = 0.29), PPG-30-buteth-30 (IOB value = 0.94), diisopropyl sebacate (IOB value = 0.4), neopentyl glycol diheptanoate (IOB value = 0.33), diethylhexyl succinate (IOB value = 0.32), isononyl isononanoate (IOB value = 0.2), isopropyl myristate (IOB value = 0.18), isoamyl laurate (IOB value = 0.18), PPG-2 myristyl propionate (IOB value = 0.26), polyglyceryl-2 triisostearate (IOB value = 0.26), polyglyceryl-2 diisostearate (IOB value = 0.41), diethoxyethyl succinate (IOB value = 1.13), dioctyl adipate (IOB value = 0.29), diisopropyl adipate (IOB value = 0.55), dioctyl sebacate (IOB value = 0.24), di-PPG-3 myristyl ether adipate (IOB value = 0.55), PPG-14 butyl ether (IOB value = 0.41), PPG-15 stearyl ether (IOB value = 0.32), PPG-3 myristyl ether (IOB value = 0.35), octyl palmitate (IOB value = 0.13), isopropyl palmitate (IOB value = 0.16), butyl stearate (IOB value = 0.14), hexyl laurate (IOB value = 0.17), myristyl myristate (IOB value = 0.11), decyl oleate (IOB value = 0.11), isotridecyl isononanoate (IOB value = 0.15), cetyl ethylhexanoate (IOB value = 0.13), pentaerythrityl tetraethylhexanoate (IOB value = 0.35), dioctyl succinate (IOB value = 0.36), glycol distearate (IOB value = 0.16), glyceryl diisostearate (IOB value = 0.29), neopentyl glycol dicaprate (IOB value = 0.25), diisostearyl malate (IOB value = 0.28), trimethylolpropane triisostearate (IOB value = 0.16), glyceryl tri-2-ethylhexanoate (triethylhexanoin) (IOB value = 0.35), trimethylolpropane trioctanoate (IOB value = 0.33), trimethylolpropane triisostearate (IOB value = 0.16), diisobutyl adipate (IOB value = 0.46), N-lauroyl-L-glutamic acid-2-octyldodecyl ester (IOB value = 0.29), 2-hexyldecyl adipate (IOB value = 0.16), ethylhexyl methoxycinnamate (IOB value = 0.28), 2-ethylhexyl palmitate (IOB value = 0.13), 2-ethylhexyl ethylhexanoate (IOB value = 0.2), triisostearin (IOB value = 0.16), PPG-3 dipivalate (IOB value = 0.52), caprylic/capric triglyceride (IOB value = 0.33), alkyl benzoate (C12-15) (IOB value = 0.18), phenethyl benzoate (IOB value = 0.3), butyloctyl salicylate (IOB value = 0.43), and neopentyl glycol diheptanoate (IOB value = 0.33), but are not limited thereto.

Examples of ultraviolet absorbers that can be regarded as polar oils include ultraviolet absorbers having an IOB of 0.10 or greater. Specific examples include, but are not limited to, organic ultraviolet absorbers such as ethylhexyl methoxycinnamate, octocrylene, polysilicone-15, t-butyl methoxydibenzoylmethane, ethylhexyl triazone, bis-ethylhexyloxyphenol methoxyphenyl triazine, diethylamino hydroxybenzoyl hexyl benzoate, benzophenone-3, methylene bis-benzotriazolyl tetramethylbutylphenol, homosalate, and ethylhexyl salicylate. These ultraviolet absorbers can be used independently or in combination of two of more.

In the present invention, the provision that the oil component comprises a liquid polar oil does not exclude other polar oils (for example, a polar oil that is solid at 25°C) or oil components other than polar oils (for example, a hydrocarbon oil or a silicone oil). As long as the effect of the present invention is not impaired, the oil component may further comprise oil components other than these liquid polar oils. For example, from the viewpoint of improving usability, the oil component can comprise at least one selected from the group consisting of hydrocarbon oils and silicone oils. Particularly, it is preferable that at least one selected from the group consisting of liquid hydrocarbon oils and liquid silicone oils be contained, and it is more preferable that both a liquid hydrocarbon oil and a liquid silicone oil be contained. Liquid hydrocarbon oil refers to a hydrocarbon oil that is liquid at 25°C, and liquid silicone oil refers to a silicone oil that is liquid at 25°C.

In the composition of the present invention, the content of the liquid polar oil in the oil component is not particularly limited, and for example, may be 10% by mass or more, 11% by mass or more, 12% by mass or more, 13% by mass or more, 14% by mass or more, or 15% by mass or more, and may be 50% by mass or less, 45% by mass or less, 40% by mass or less, 35% by mass or less, 30% by mass or less, 25% by mass or less, 20% by mass or less, or 15% by mass or less, relative to the entire oil component.

### (Hydrocarbon oil)

In the composition of the present invention, the oil component may further comprise a hydrocarbon oil. Hydrocarbon oil refers to a hydrocarbon oil other than the polar oil according to the present invention.

The hydrocarbon oil may be a volatile hydrocarbon oil, or may be a non-volatile hydrocarbon oil.

In the present invention, an oil component being "volatile" refers to an oil component that exhibits a volatile content of more than 20% when left to stand at 30°C for 120 min under atmospheric pressure. Correspondingly, in the present invention, an oil component being "non-volatile" refers to an oil component that exhibits a volatile content of 20% or less when left to stand at 30°C for 120 min under atmospheric pressure. The volatility described above can be evaluated by the following method. 2 mg/cm² of an oil component is applied dropwise onto a 5-mm square acrylic resin substrate, and the weights before and after standing at 30°C for 120 min are measured on a precision balance. From the difference in weight before and after standing for 120 min, the amount lost due to volatilization of the oil component is calculated, and the ratio (%) relative to the weight at application is evaluated.

Specific examples of the hydrocarbon oil include, but are not limited to, liquid paraffin, hydrogenated polydecene, decane, dodecane, isododecane, isohexadecane, squalane, squalene, and mixtures of two or more thereof. Among these hydrocarbon oils, a liquid hydrocarbon oil is preferably used.

The content of the hydrocarbon oil, if the oil component in the present invention comprises a hydrocarbon oil, is not particularly limited, and for example, may be 10% by mass or more, 15% by mass or more, 20% by mass or more, 25% by mass or more, 30% by mass or more, 35% by mass or more, 40% by mass or more, 45% by mass or more, or 50% by mass or more, and may be 80% by mass or less, 75% by mass or less, 70% by mass or less, 65% by mass or less, 60% by mass or less, 55% by mass or less, 50% by mass or less, 45% by mass or less, 40% by mass or less, 35% by mass or less, 30% by mass or less, 25% by mass or less, 20% by mass or less, or 15% by mass or less, relative to the entire oil component.

### (Silicone oil)

Silicone oil refers to an oil component, among oil components usable in cosmetics, having a main backbone formed by siloxane bonds. Substances that "has a main backbone formed by siloxane bonds" and can be used as an "ultraviolet absorber" described above are excluded from the "silicone oil" referred herein.

In the present invention, the silicone oil may be a volatile silicone oil, or may be a non-volatile silicone oil.

Specific examples of the silicone oil include, but are not limited to, linear silicone oils such as polydimethylsiloxane (dimethicone), methylphenyl polysiloxane, and methylhydrogenpolysiloxane; cyclic silicone oils such as octamethylcyclotetrasiloxane, decamethylcyclopentasiloxane, and dodecamethylcyclohexasiloxane; and combinations of two or more thereof. Among these silicone oils, a liquid silicone oil is preferably used.

The content of the silicone oil, if the oil component in the present invention comprises a silicone oil, is not particularly limited, and for example, may be 10% by mass or more, 15% by mass or more, 20% by mass or more, 25% by mass or more, 30% by mass or more, 35% by mass or more, 40% by mass or more, 45% by mass or more, or 50% by mass or more, and may be 80% by mass or less, 75% by mass or less, 70% by mass or less, 65% by mass or less, 55% by mass or less, 50% by mass or less, 45% by mass or less, 40% by mass or less, 35% by mass or less, 30% by mass or less, 25% by mass or less, 20% by mass or less, or 15% by mass or less, relative to the entire oil component.

### <Oil-phase thickener>

The composition of the present invention comprises an oil-phase thickener. Oil-phase thickener refers to a substance that thickens or solidifies an oil component so as to prevent the oil component from flowing away.

In the present invention, the content of the oil-phase thickener is not particularly limited. For example, the oil-phase thickener, relative to 100 parts by mass in total of the compound I, the oil component, and the oil-phase thickener, may be 0.1 parts by mass or more, 0.5 parts by mass or more, 1.0 parts by mass or more, 1.5 parts by mass or more, 2.0 parts by mass or more, 2.5 parts by mass or more, 3.0 parts by mass or more, 3.5 parts by mass or more, 4.0 parts by mass or more, 4.5 parts by mass or more, 5.0 parts by mass or more, 5.5 parts by mass or more, 6.0 parts by mass or more, 6.5 parts by mass or more, 7.0 parts by mass or more, 7.5 parts by mass or more, 8.0 parts by mass or more, 8.5 parts by mass or more, 9.0 parts by mass or more, or 10 parts by mass or more, and may be 15 parts by mass or less, 14 parts by mass or less, 13 parts by mass or less, 12 parts by mass or less, 11 parts by mass or less, 10 parts by mass or less, 9.0 parts by mass or less, 8.0 parts by mass or less, 7.0 parts by mass or less, 6.0 parts by mass or less, or 5.0 parts by mass or less.

In the present invention, the oil-phase thickener comprises at least one selected from waxes and oil gelling agents having a saccharide backbone, an amide bond, or a urethane bond. Amide bond refers to a carbon-nitrogen bond formed by dehydration condensation of a carboxylic acid and an amine, and can be represented by, for example, -C(=O)-N(Rₓ)- (where in the formula, Rₓ may be a hydrogen atom, or may be another substituent such as an alkyl group). Urethane bond refers to a bond that occurs in a reaction between an isocyanate group and a hydroxyl group, and can be represented by, for example, -O-C(=O)-N(R_{y})- (where in the formula, R_{y} may be a hydrogen atom, or may be another substituent such as an alkyl group).

More specifically, the wax may be at least one selected from the group consisting of microcrystalline wax, polyethylene, candelilla wax, sunflower seed wax, paraffin, synthetic wax, beeswax, Japan wax, rice bran wax, carnauba wax, and ceresin.

The oil gelling agent, for example, may be at least one selected from the group consisting of dibutyl lauroyl glutamide, polyamide-8, polyurethane-79, dextrin palmitate, dextrin (palmitate/ethylhexanoate), dibutyl ethylhexanoyl glutamide, and dextrin myristate. Among these oil gelling agents, for example, from the viewpoint of easily contributing to a three-dimensional network structure, oil gelling agents having a branched chain or a substituent (preferably a bulky substituent) in the molecule are preferable, and oil gelling agents having a hydroxy group, an amide bond, or a urethane bond for network formation are particularly preferable.

### <<Cosmetic>>

The composition of the present invention described above can be blended into various cosmetics. Specifically, the present invention can provide an oil-based cosmetic comprising the composition of the present invention, an oil-based solid cosmetic comprising the composition of the present invention, and a water-in-oil emulsion cosmetic comprising the composition of the present invention.

The composition of the present invention can suppress crystallization of the above compound I over time, and exhibits satisfactory usability. Therefore, these cosmetics comprising the composition of the present invention similarly exhibits satisfactory usability.

In addition to the composition of the present invention described above, these cosmetics can be appropriately blended with various other components, in ranges that do not affect the effect of the present invention. The necessity for blending and the blending amounts of such other components may be appropriately adjusted according to the type of cosmetic described above.

The various other components are not particularly limited, and may be components that can generally be blended into cosmetics. Examples can include water, thickeners other than the oil-phase thickeners described above, surfactants, moisturizers, stabilizers, water-soluble polymers, oil-soluble polymers, film-forming agents, higher fatty acids, sequestering agents, lower alcohols, higher alcohols, various extracts, saccharides, amino acids, organic amines, polymer emulsions, chelating agents, ultraviolet scattering agents, various powders, pH adjusters, skin nutrients, vitamins; water-soluble chemical agents that can be applied to pharmaceuticals, quasi-drugs, and cosmetics; buffers, anti-fading agents, preservatives, dispersants, propellants, colorants, and fragrances.

### <Oil-based cosmetic>

In the present invention, oil-based cosmetic refers to a single-phase, oil-phase cosmetic. Oil-based cosmetics, generally, are substantially free of water, which is present separately from the oil phase.

In the oil-based cosmetic of the present invention, the content of the composition of the present invention is not particularly limited, and for example, may be 70% by mass or more, 75% by mass or more, 80% by mass or more, 85% by mass or more, 90% by mass or more, 95% by mass or more, or 99% by mass or more, and may be 100% by mass or less, 99% by mass or less, 98% by mass or less, 97% by mass or less, 96% by mass or less, or 95% by mass or less.

### <Oil-based solid cosmetic>

In the present invention, oil-based solid cosmetic refers to an oil-based cosmetic, among the oil-based cosmetics described above, that is solid at 25°C. Therefore, the content of the composition of the present invention in the oil-based solid cosmetic of the present invention may be the same as in the oil-based cosmetics described above.

### <Water-in-oil emulsion cosmetic>

The water-in-oil emulsion cosmetic of the present invention may comprise in the continuous phase (external phase) thereof the composition of the present invention.

In the water-in-oil emulsion cosmetic of the present invention, the content of the composition of the present invention is not particularly limited, and for example, may be 20% by mass or more, 25% by mass or more, 30% by mass or more, 35% by mass or more, 40% by mass or more, 45% by mass or more, 50% by mass or more, 55% by mass or more, 60% by mass or more, 65% by mass or more, 70% by mass or more, 75% by mass or more, 80% by mass or more, 85% by mass or more, or 90% by mass or more, and may be 90% by mass or less, 85% by mass or less, 80% by mass or less, 75% by mass or less, 70% by mass or less, 65% by mass or less, 60% by mass or less, or 55% by mass or less.

In the water-in-oil emulsion cosmetic of the present invention, the content of water is not particularly limited, and for example, may be 5.0% by mass or more, 10% by mass or more, 15% by mass or more, 20% by mass or more, 25% by mass or more, or 30% by mass or more, and may be 65% by mass or less, 60% by mass or less, 55% by mass or less, 50% by mass or less, 45% by mass or less, 40% by mass or less, 35% by mass or less, 30% by mass or less, or 25% by mass or less.

### <Dosage form>

Examples of dosage forms of the cosmetic comprising the composition of the present invention include, but are not limited to, solid, semi-solid, solid paste, stick, emulsion, liquid, gel, sherbet, cream, ointment, and paste.

### <Application>

The composition of the present invention can be suitably used as various cosmetics or raw materials thereof.

For example, the composition of the present invention, by comprising the compound I, exhibits absorption capacity in both the UVA region and the UVB region and exhibits ultraviolet absorption capabilities in a broad range of wavelengths. Therefore, the present invention can provide a sunscreen cosmetic comprising the composition of the present invention.

Additionally, examples of applications of the composition of the present invention include, but are not limited to, cosmetic primer, foundation, blush, lipstick, and lip cream.

### EXAMPLES

The present invention will further be described in detail with reference to the Examples below. However, the present invention is not limited thereto. Hereinafter, unless specified otherwise, contents are expressed in % by mass.

### <<Examples 1 to 10 and Comparative Examples 1 to 3>>

Oil-based cosmetic compositions of Examples 1 to 10 and Comparative Examples 1 to 3 were prepared based on the formulations in Table 1. More specifically, all raw materials were heated to 90°C or higher, mixed, and then cooled to room temperature to obtain each composition. Each of the compositions of Examples 1 to 10 and Comparative Examples 2 and 3 was solid (stick form), whereas the composition of Comparative Example 1 was liquid.

As the compound I, a compound represented by the structural formula below was used.

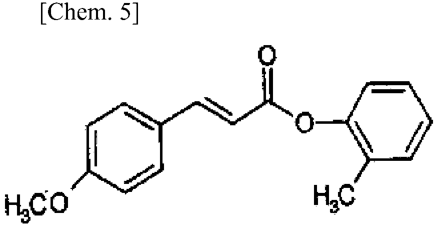

Each cosmetic was confirmed to have formed a single transparent layer in the heating step during preparation. For each composition after preparation, the presence or absence of crystal precipitation after standing at room temperature (25°C) for 4 days and after standing for 1 week was confirmed by both visual inspection and application, based on the following criteria. Results are shown in Table 1.

### <Evaluation criteria of presence or absence of crystal precipitation>

"A": No crystal precipitation was observed in visual inspection or application.
"B": Partial crystal precipitation was observed in visual inspection, but was not perceived upon application.
"C": Crystal precipitation could be confirmed in visual inspection, and grittiness was perceived upon application.
"D": Crystal precipitation could be confirmed in visual inspection, and crystal precipitation was more extensive than in "C" above. Grittiness was also perceived upon application.

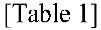

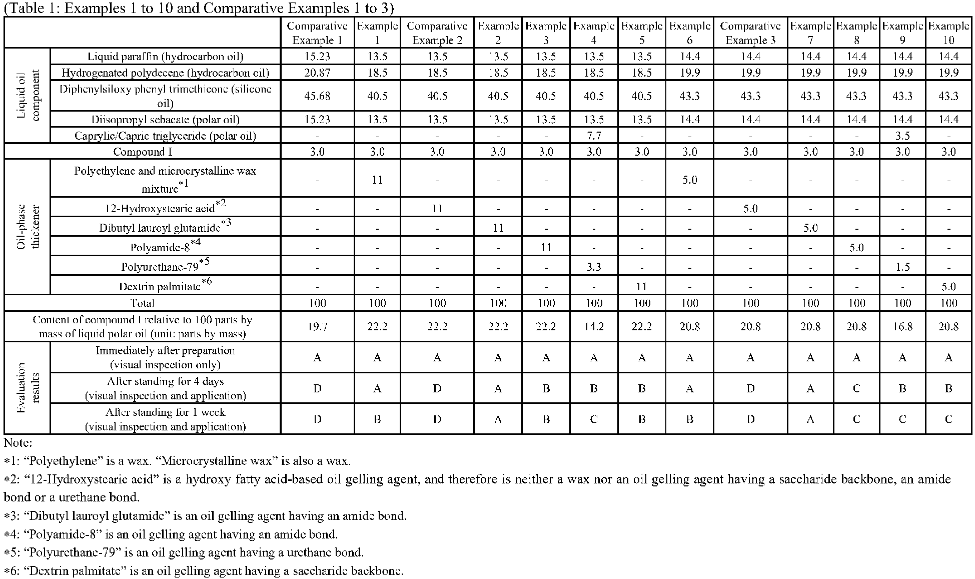

As is apparent from the results in Table 1, the composition of Comparative Example 1, which comprised no oil-phase thickener, exhibited no precipitation of crystals of the compound I immediately after preparation. However, extensive crystal precipitation was confirmed after standing for 4 days, and grittiness was perceived at the time of application.

It was found that in all of the compositions of Examples 1 to 10, crystal precipitation of the compound I was suppressed not only immediately after preparation but also after standing for 4 days and after standing for 1 week.

The results suggested that the compositions of Examples 1 to 3, 5 to 8, and 10 in particular can suppress the precipitation of crystals of the compound I over time while maintaining the same amount or a smaller amount of liquid polar oil as in the composition of Comparative Example 1, thereby exhibiting satisfactory usability.

In Comparative Examples 2 and 3, although an oil-phase thickener was blended in, the oil-phase thickener was a hydroxy fatty acid-based oil gelling agent and was not selected from the group consisting of waxes and oil gelling agents having a saccharide backbone, an amide bond, or a urethane bond, and thus was found to be incapable of suppressing the precipitation of crystals of the compound I over time.

### <<Examples 11 to 14 and Comparative Examples 4 to 7>>

In the same manner as in Example 1 above, solid oil-based cosmetic composition (stick form) of Examples 11 to 14 and Comparative Examples 4 to 7 were prepared based on the formulations in Table 2 and evaluated. Evaluation results are shown in Table 2.

**[Table 2]**

| (Example 2: Examples 11 to 14 and Comparative Examples 4 to 7) | | | | | | | | | |
|---|---|---|---|---|---|---|---|---|---|
| | | Comparative Example 4 | Comparative Example 5 | Comparative Example 6 | Comparative Example 7 | Example 11 | Example 12 | Example 13 | Example 14 |
| Liquid oil component | Liquid paraffin (hydrocarbon oil) | 13.5 | 14.4 | 13.5 | 14.4 | 13.5 | 14.4 | 13.5 | 14.4 |
| | Hydrogenated polydecene (hydrocarbon oil) | 18.5 | 19.9 | 18.5 | 19.9 | 18.5 | 19.9 | 18.5 | 19.9 |
| | Diphenylsiloxy phenyl trimethicone (silicone oil) | 40.5 | 43.3 | 40.5 | 43.3 | 40.5 | 43.3 | 40.5 | 43.3 |
| | Diisopropyl sebacate (polar oil) | 13.5 | 14.4 | 13.5 | 14.4 | 13.5 | 14.4 | 13.5 | 14.4 |
| Compound I | | 3.0 | 3.0 | 3.0 | 3.0 | 3.0 | 3.0 | 3.0 | 3.0 |
| Oil-phase thickener | Stearic acid*¹ | 11 | 5.0 | - | - | - | - | - | - |
| | Behenic acid*² | - | - | 11 | 5.0 | - | - | - | - |
| | Polyethylene*³ | - | - | - | - | 11 | 5.0 | - | - |
| | Microcrystalline wax*⁴ | - | - | - | - | - | - | 11 | 5.0 |
| Total | | 100 | 100 | 100 | 100 | 100 | 100 | 100 | 100 |
| Content of compound I relative to 100 parts by mass of liquid polar oil (unit: parts by mass) | | 22.2 | 20.8 | 22.2 | 20.8 | 22.2 | 20.8 | 22.2 | 20.8 |
| Evaluation results | Immediately after preparation (visual inspection only) | A | A | A | A | A | A | A | A |
| | After standing for 4 days (visual inspection and application) | D | D | D | D | A | A | A | A |
| | After standing for 1 week (visual inspection and application) | D | D | D | D | B | B | B | B |

| | | | | | | | | | |
|---|---|---|---|---|---|---|---|---|---|
| Note: *1: "Stearic acid" is a fatty acid-based oil gelling agent, and therefore is neither a wax nor an oil gelling agent having a saccharide backbone, an amide bond or a urethane bond. *2: "Behenic acid" is a fatty acid-based oil gelling agent, and therefore is neither a wax nor an oil gelling agent having a saccharide backbone, an amide bond or a urethane bond. *3: "Polyethylene" is a wax. *4: "Microcrystalline wax" is a wax. | | | | | | | | | |

As is apparent from the results in Table 2, it was found that in all of the compositions of Examples 11 to 14, crystal precipitation of the compound I was suppressed not only immediately after preparation but also after standing for 4 days and after standing for 1 week.

The compositions of Comparative Examples 4 to 7 all exhibited no precipitation of crystals of the compound I immediately after preparation. However, extensive crystal precipitation was confirmed after standing for 4 days, and grittiness was perceived at the time of application.

### <<Examples 15 to 17, Comparative Example 8, and Reference Example 1>>

Cosmetic compositions of Examples 15 to 17, Comparative Example 8, and Reference Example 1 were prepared based on the formulations in Table 3. Based on the above criteria, the presence or absence of crystal precipitation was confirmed under the standing conditions as follows. Results are shown in Table 3.

### (Standing conditions)

- Standing at room temperature (25°C) for 1 week after preparation, and continuing to stand until 2 months after preparation
- Standing at 0°C for 1 month after preparation

**[Table 3]**

| (Table 3: Examples 15 to 17, Comparative Example 8, and Reference Example 1) | | | | | | |
|---|---|---|---|---|---|---|
| | | Comparative Example 8 | Reference Example 1 | Example 15 | Example 16 | Example 17 |
| Liquid oil component | Liquid paraffin (hydrocarbon oil) | - | 10 | 10 | 10 | 11 |
| | Hydrogenated polydecene (hydrocarbon oil) | - | 13.45 | 13.45 | 13.45 | 11.45 |
| | Diphenylsiloxy phenyl trimethicone (silicone oil) | - | 30 | 30 | 30 | 30 |
| Liquid polar oil (including ultraviolet absorber) | Diisopropyl sebacate | 95 | 10 | 10 | 10 | 10 |
| | Caprylic/Capric triglyceride | - | - | - | - | 0.7 |
| | Homosalate | - | 10 | 10 | 10 | 10 |
| | Octyl methoxycinnamate | - | 3.0 | - | - | - |
| | Octocrylene | - | 5.0 | 5.0 | 5.0 | 5.0 |
| Solid ultraviolet absorber | t-Butyl methoxydibenzoylmethane | - | 3.0 | 3.0 | 3.0 | 3.0 |
| | Bis-ethylhexyloxyphenol methoxyphenyl triazine | - | 2.0 | 2.0 | 2.0 | 2.0 |
| | Diethylamino hydroxybenzoyl hexyl benzoate | - | 2.0 | 2.0 | 2.0 | 2.0 |
| Stabilizer | BHT | - | 0.05 | 0.05 | 0.05 | 0.05 |
| Compound I | | 5.0 | - | 3.0 | 3.0 | 3.0 |
| Oil-phase thickener | Hydroxystearic acid | - | 7.5 | 7.5 | 7.5 | 7.5 |
| | Dibutyl lauroyl glutamide | - | 2.0 | 2.0 | 2 | 2.0 |
| | Polyamide-8 | - | 2.0 | 2.0 | - | 2.0 |
| | Polyurethane-79 | - | - | - | - | 0.3 |
| | Dextrin palmitate | - | - | - | 2.0 | - |
| | Total | 100 | 100 | 100 | 100 | 100 |
| Content of compound I relative to 100 parts by mass of liquid polar oil (unit: parts by mass) | | 5.2 | - | 12.0 | 12.0 | 11.7 |
| Evaluation results | Immediately after preparation (visual inspection only) | A | A | A | A | A |
| | Room temperature for 1 week (visual inspection and application) | D | A | A | A | A |
| | 0°C for 1 month (visual inspection and application) | D | A | A | A | A |
| | Room temperature for 2 months (visual inspection and application) | D | A | A | A | B |

As is apparent from the results in Table 3, the composition of Comparative Example 8, which comprised no oil-phase thickener, exhibited no precipitation of crystals of the compound I immediately after preparation. However, it was found that after standing at room temperature for 1 week, standing at 0°C for 1 month, or standing at room temperature for 2 months, extensive crystal precipitation was confirmed, and grittiness was perceived upon application.

It was found that in all of the compositions of Examples 15 to 17, crystal precipitation of the compound I was suppressed not only immediately after preparation but also after standing at room temperature for 1 week, standing at 0°C for 1 month, and standing at room temperature for 2 months.

As is apparent from the results of Reference Example 1, when the compound I was not blended, no crystal precipitation was observed.

## Claims

1. A cosmetic composition, comprising
a compound I having a structure of formula (I) below: 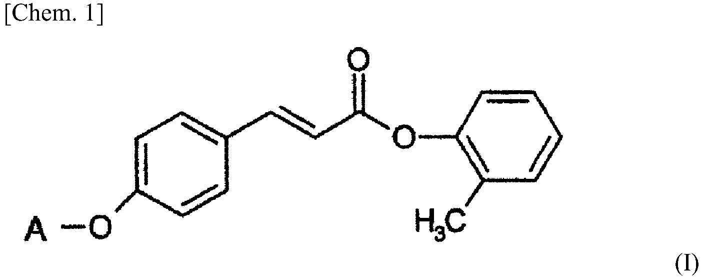 where in the formula, -OA represents an alkoxy group;
an oil component comprising a liquid polar oil; and
an oil-phase thickener,
wherein the compound I is dissolved in the oil component,
wherein the compound I, relative to 100 parts by mass of the liquid polar oil, is 2.8 parts by mass or more, and
wherein the oil-phase thickener comprises at least one selected from a group consisting of waxes and oil gelling agents having a saccharide backbone, an amide bond, or a urethane bond.

2. The cosmetic composition according to claim 1, wherein the wax is at least one selected from a group consisting of microcrystalline wax, polyethylene, candelilla wax, sunflower seed wax, paraffin, synthetic wax, beeswax, Japan wax, rice bran wax, carnauba wax, and ceresin.

3. The cosmetic composition according to claim 1 or 2, wherein the oil gelling agent is at least one selected from a group consisting of dibutyl lauroyl glutamide, polyamide-8, polyurethane-79, dextrin palmitate, dextrin (palmitate/ethylhexanoate), dibutyl ethylhexanoyl glutamide, and dextrin myristate.

4. The cosmetic composition according to any one of claims 1 to 3, wherein the oil-phase thickener, relative to 100 parts by mass in total of the compound I, the oil component, and the oil-phase thickener, is 0.1 parts by mass or more and 15 parts by mass or less.

5. The cosmetic composition according to any one of claims 1 to 4, wherein content of the liquid polar oil in the oil component is 10% by mass or more.

6. The cosmetic composition according to any one of claims 1 to 5, wherein the compound I, relative to 100 parts by mass in total of the compound I, the oil component, and the oil-phase thickener, is 3.0 parts by mass or more.

7. An oil-based cosmetic comprising the cosmetic composition according to any one of claims 1 to 6.

8. An oil-based solid cosmetic comprising the cosmetic composition according to any one of claims 1 to 6.

9. A water-in-oil emulsion cosmetic comprising the cosmetic composition according to any one of claims 1 to 6.

10. A sunscreen cosmetic comprising the cosmetic composition according to any one of claims 1 to 6.
